# EUROPEAN PATENT APPLICATION

(11) **EP 3 557 587 A1**
(43) Date of publication of application: **23.10.2019**
(21) Application number: 18168165.1
(22) Date of filing: 19.04.2018
(51) Int. Cl.: G16H 20/60

(54) **AN APPARATUS AND METHOD FOR PERSONALIZED MEAL PLAN GENERATION**

(71) Applicant: Koninklijke Philips N.V., 5656 AE Eindhoven (NL)
(72) Inventor: XIAO, Weimin, 5656 AE Eindhoven (NL); LUO, ZhongChi, 5656 AE Eindhoven (NL); WIJNOLTZ, Anna Louise, 5656 AE Eindhoven (NL); LU, Weihua, 5656 AE Eindhoven (NL); JIN, Yafang, 5656 AE Eindhoven (NL)
(74) Representative: de Haan, Poul Erik

(57) **Abstract**

There is provided a computer-implemented method for generating a personalized meal plan for a subject. The method comprises acquiring (202) data associated with the subject, generating (204) a target nutrient value for the subject for a nutrient type based on the acquired data and selecting (206) a plurality of recommended food ingredients based on the generated value, generating (208) a meal plan by selecting a recipe based on the plurality of selected recommended food ingredients, determining (210) a difference between a provided amount of the nutrient type by the selected recipe and the generated target nutrient value for the nutrient type, and adjusting (212) the meal plan based on the determined difference.

## Description

### FIELD OF THE INVENTION

The present disclosure relates to an apparatus and method for generating a personalized meal plan for a subject.

### BACKGROUND OF THE INVENTION

A good diet is important to a person's health and well-being. In order to help people consume meals with balanced nutrition, there are currently many applications and solutions available that have been developed to recommend meal plans or recipes to users. Typically, these applications or solutions aim to provide a recommended meal plan or recipe based on personal information and raw nutrient data. For example, a daily caloric intake amount can be assigned to a user based on a standardized formula or chart taking into account of factors such as age, gender, height, and body weight. A corresponding meal plan or recipe would be recommended by, for example, satisfying the daily caloric intake amount of the user based on the raw nutrient data.

### SUMMARY OF THE INVENTION

As there are increasingly more dietary requirements and approaches that can be adopted, the factors and formulae used to recommend corresponding meal plans or recipes have become more complex. It is therefore important to provide accurate recommendation of meal plans or recipes to ensure that the health objectives of the users are achieved. However, the applications and solutions currently available provide recommendation or personalization based on nutrient data of food ingredients in pro-processed (e.g. raw) states. This means that the change in nutrients in the food ingredients during a food preparation process (e.g. cooking) is not taken into account. For example, during a food preparation process there may be nutrient leaching into water (e.g. during boiling), dripping oil, heat degradation, oxygen degradation, light-induced degradation, and enzyme degradation, etc. which causes nutrient change or nutrient loss. Moreover, there are other factors, such as those from the surrounding environment of the nutrients (e.g. a pH value and a state of the food ingredient - such as whether it is solid state and/or whether it is enclosed) will also play a role in the extent of change (e.g. degradation) of the nutrients.

Accordingly, one of the ways to improve accuracy of personalizing or recommendation of meal plans or recipes is to consider nutrient change or nutrient loss occurring during food preparation.

As noted above, there are a number of disadvantages associated with the currently available method for generating a personalized meal plan for a subject. It would therefore be advantageous to provide an improved method for generating a meal plan for a subject.

To better address one or more of the concerns mentioned earlier, in a first aspect, a computer-implemented method for generating a personalized meal plan for a subject, the method comprising: acquiring data associated with the subject; generating a target nutrient value for the subject for a nutrient type based on the acquired data; selecting a plurality of recommended food ingredients based on the generated target nutrient value; generating a meal plan by selecting a recipe stored in one or more databases based on the plurality of selected recommended food ingredients, wherein the selected recipe comprises a respective amount of each of a plurality of required food ingredients and food preparation instructions; determining a difference between a provided amount of the nutrient type by the selected recipe and the generated target nutrient value for the nutrient type, based on the amount of each of the plurality of required food ingredients and the food preparation instructions in the selected recipe; and adjusting the meal plan based on the determined difference.

In some embodiments, adjusting the meal plan may comprise changing at least one of: at least one of the plurality of required food ingredients, the amount of at least one of the plurality of required food ingredients, and the food preparation instructions.

In some embodiments, adjusting the meal plan may be performed so as to minimize the difference between the provided amount of the nutrient type and the generated target nutrient value for the nutrient type.

In some embodiments, the meal plan may be adjusted if the determined difference exceeds a predetermined threshold.

In some embodiments, the target nutrient value may represent a recommended amount of the nutrient type to be consumed by the subject within a predetermined time period, and selecting the plurality of recommended food ingredients may further be based on at least one previously recommended food ingredient for the subject before the predetermined time period.

In some embodiments, the method further may further comprise generating a respective recommended amount to be consumed by the subject for each of the plurality of selected food ingredients. In these embodiments, selecting the recipe may further be based on at least one of the recommended amounts for the plurality of selected food ingredients.

In some embodiments, generating the meal plan may comprise: acquiring a plurality of candidate recipes from the one or more databases based on the plurality of recommended food ingredients; and selecting one of the candidate recipes based on a user input.

In some embodiments, selecting the plurality of recommended food ingredients may comprise: acquiring a plurality of candidate food ingredients from one or more databases based on the generated target nutrient value; and selecting a plurality of recommended food ingredients from the plurality of candidate food ingredients based on a user input.

In some embodiments, determining a difference between a provided amount of the nutrient type by the selected recipe and the generated target nutrient value for the nutrient type may be based on a retention rate of the nutrient type associated with at least one of the required food ingredients in the generated meal plan.

In some embodiments, determining a difference between a provided amount of the nutrient type by the selected recipe and the generated target nutrient value for the nutrient type may be based on data associated with a pre-processed state of at least one of the required food ingredients in the generated meal plan.

In some embodiments, the data associated with the subject may comprise information relating to at least one of: the gender of the subject, the age of the subject, the weight of the subject, the height of the subject, physical activity of the subject, a metabolic rate of the subject, a health target of the subject, and a food preference of the subject.

In some embodiments, the nutrient type may be one of: total energy, a macronutrient, a micronutrient.

In a second aspect, there is provided a computer program product comprising a computer readable medium, the computer readable medium having computer readable code embodied therein, the computer readable code being configured such that, on execution by a suitable computer or processor, the computer or processor is caused to perform the method according to the first aspect.

In a third aspect, there is provided an apparatus for generating a personalized meal plan for a subject, the apparatus comprising a processor configured to: acquire data associated with the subject, wherein the acquired data comprises at least a food preference of the subject; generate a target nutrient value for the subject for a nutrient type based on the acquired data; select a plurality of recommended food ingredients based on the generated target nutrient value and the food preference of the subject; generate a meal plan by selecting a recipe stored in one or more databases based on the plurality of selected recommended food ingredients, wherein the selected recipe comprises a respective amount of each of a plurality of required food ingredients, and food preparation instructions; determine a difference between a provided amount of the nutrient type by the selected recipe and the generated target nutrient value for the nutrient type, based on the amount of each of the plurality of required food ingredients and the food preparation instructions in the selected recipe; and adjust the meal plan based on the determined difference.

According to the aspects and embodiments described above, the limitations of existing techniques are addressed. In particular, the above-described aspects and embodiments enable a personalized meal plan that considers nutrient loss caused by food preparation techniques or methods to be generated. The generation of the meal plan takes into account nutrient loss caused by different food preparation technique or methods. In this way, the subject is able to consume an accurate ideal amount of food in order to achieve a desired nutrient intake target. There is thus provided an improved method and apparatus for generating a meal plan for a subject.

These and other aspects of the disclosure will be apparent from and elucidated with reference to the embodiment(s) described hereinafter.

### BRIEF DESCRIPTION OF THE DRAWINGS

For a better understanding of the embodiments, and to show more clearly how they may be carried into effect, reference will now be made, by way of example only, to the accompanying drawings, in which:
Fig. 1 is a block diagram of an apparatus for generating a personalized meal plan for a subject according to an embodiment; and
Fig. 2 illustrates a method for generating a personalized meal plan for a subject according to an embodiment.

### DETAILED DESCRIPTION OF EMBODIMENTS

As noted above, there is provided an improved apparatus and a method of operating the same which addresses the existing problems.

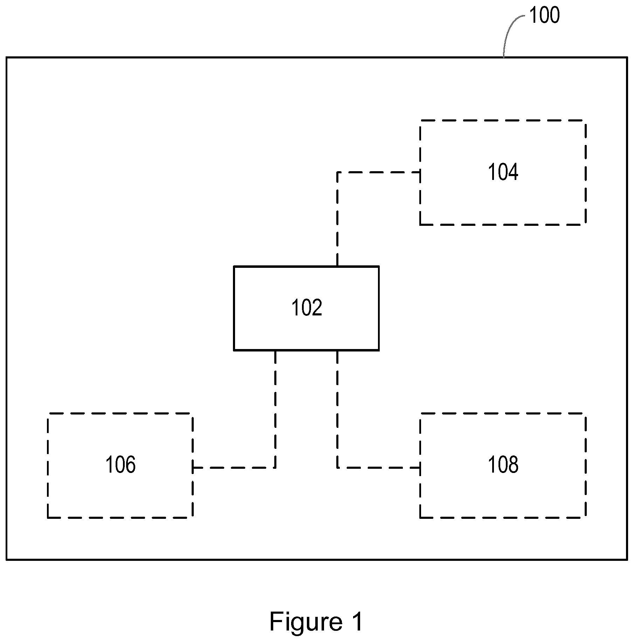

Fig. 1 shows a block diagram of an apparatus 100 according to an embodiment, which can be used for generating a personalized meal plan for a subject. A meal plan comprises at least one recipe which contain instructions for a user on how to prepare a meal for the subject. In some embodiments, a recipe may comprise a respective amount of each of a plurality of food ingredients required (and to be consumed by the subject) and food preparation instructions, such as "oven bake 400g of potatoes for 15 minutes".

As illustrated in Fig. 1, the apparatus comprises a processor 102 that controls the operation of the apparatus 100 and that can implement the method described herein. The processor 102 can comprise one or more processors, processing units, multi-core processor or modules that are configured or programmed to control the apparatus 100 in the manner described herein. In particular implementations, the processor 102 can comprise a plurality of software and/or hardware modules that are each configured to perform, or are for performing, individual or multiple steps of the method described herein.

Briefly, the processor 102 is configured to acquire data associated with the subject, and generate a target nutrient value for the subject for a nutrient type based on the acquired data. A nutrient type may be at least one of: a total energy, a macronutrient, and a micronutrient. For example, a nutrient type may be a type of macronutrient such as carbohydrate, protein, lipids, and dietary fiber or a type of micronutrient such as vitamin C and sodium. The target nutrient value for the subject for a nutrient type may represent a recommended amount of the nutrient type that the subject should consume from a meal and/or within a predetermined time period.

Based on the generated target nutrient value, a plurality of recommended food ingredients is selected. Then, based on the plurality of selected recommended food ingredients, a meal plan is generated by selecting a recipe stored in one or more databases. The selected recipe comprises a respective amount of each of a plurality of required food ingredients and food preparation instructions.

The processor 102 is also configured to determine a between a provided amount of the nutrient type by the selected recipe and the generated target nutrient value for the nutrient type, based on the amount of each of the plurality of required food ingredients and the food preparation instructions in the selected recipe. The processor 102 is further configured to adjust the meal plan based on the determined difference.

In some embodiments, the apparatus 100 may further comprise at least one user interface 104. Alternative or in addition, at least one user interface 104 may be external to (i.e. separate to or remote from) the apparatus 100. For example, at least one user interface 104 may be part of another device. A user interface 104 may be for use in providing a user of the apparatus 100 with information resulting from the method described herein. For example, the processor 102 may be configured to control one or more user interfaces 104 to render (or output or display) the adjusted meal plan for the subject. Alternatively or in addition, a user interface 104 may be configured to receive a user input. For example, a user interface 104 may allow a user of the apparatus 100 to manually enter instructions, data, or information. In these embodiments, the processor 102 maybe configured to acquire the user input from one or more user interfaces 104.

A user interface 104 may be any user interface that enables the rendering (or output or display) of information to a user of the apparatus 100. Alternatively or in addition, a user interface 104 may be any user interface that enables a user of the apparatus 100 to provide a user input, interact with and/or control the apparatus 100. For example, the user interface 104 may comprise one or more switches, one or more buttons, a keypad, a keyboard, a touch screen or an application (for example, on a tablet or smartphone), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

In some embodiments, the apparatus 100 may comprise a memory 106. Alternatively or in addition, one or more memories 106 maybe external to (i.e. separate to or remote from) the apparatus 100. For example, one or more memories 106 may be part of another device. A memory 106 can be configured to store program code that can be executed by the processor 102 to perform the method described herein. A memory can be used to store information, data, signals and measurements acquired or made by the processor 102 of the apparatus 100. For example, a memory 106 maybe used to store (for example, in a local file) a plurality of recipes to be selected. The processor 102 may be configured to control a memory 106 to store the plurality of recipes.

In some embodiments, the apparatus 100 may comprise a communications interface (or circuitry) 108 for enabling the apparatus 100 to communicate with any interfaces, memories and/or devices that are internal or external to the apparatus 100. The communications interface 108 may communicate with any interfaces, memories and/or devices wirelessly or via a wired connection. For example, the communications interface 108 may communicate with one or more user interfaces 104 wirelessly or via a wired connection. Similarly, the communications interface 108 may communicate with the one or more memories 106 wirelessly or via a wired connection.

It will be appreciated that Fig. 1 only shows the components required to illustrate an aspect of the apparatus 100 and, in a practical implementation, the apparatus 100 may comprise alternative or additional components to those shown.

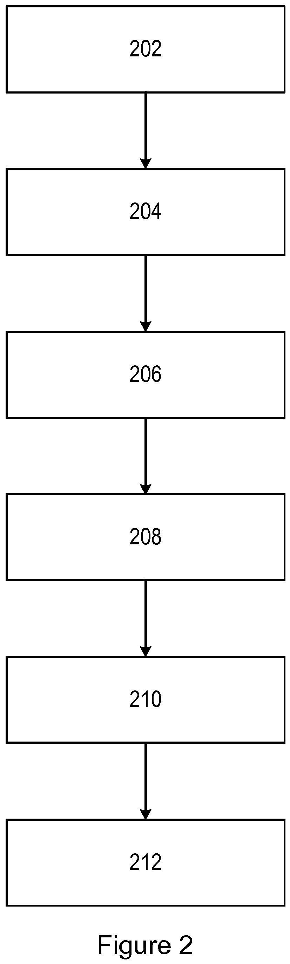

Fig. 2 illustrates a computer-implemented method for generating a care plan for a subject according to an embodiment. The illustrated method can generally be performed by or under the control of processor 102 of the apparatus 100.

With reference to Fig. 2, at block 202, data associated with the subject is acquired. More specifically, the data associated with the subject may be acquired by the processor 102 of the apparatus 100. In some embodiments, the data associated with the subject may be acquired from one or more databases in a memory 106, which may be a memory of the apparatus 100 or a memory external to the apparatus 100. The acquired data associated with the subject may comprise information relating to at least one of: the gender of the subject, the age of the subject, the weight of the subject, the height of the subject, physical activity of the subject (e.g. an amount of physical exercises performed by the subject, a number of steps taken by the subject), a health target of the subject (e.g. a target body weight, a target amount of physical exercise to be performed, etc.), and a food preference of the subject (e.g. food allergies and specific diets such as vegetarian, kosher, etc.)

Returning back to Fig. 2, at block 204, a target nutrient value for the subject for a nutrient type is generated based on the acquired data. The target nutrient value for the subject for a nutrient type may be generated by the processor 102, and it may represent a recommended amount of the nutrient type that the subject should consume from a meal and/or within a predetermined time period. In some embodiments, the target nutrient value may represent a recommended amount of the nutrient type to be consumed by the subject within a predetermined time period (e.g. a day).

For example, in some embodiments, the acquired data may at block 204 may comprise information relating to the gender of the subject, the body weight of the subject, and the physical activity of the subject. In these embodiments, the processor 102 of the apparatus 100 may generate a target nutrient value for the subject for a total amount of energy (caloric intake) to be consumed by the subject based on a standard daily energy allowance of a person of the gender and the body weight same as those of the subject, minus the energy expenditure caused by the physical exercise performed by the subject.

Returning back to Fig. 2, at block 206, a plurality of recommended food ingredients is selected. The plurality of recommended food ingredients are selected based on the generated target nutrient value at block 204.

As mentioned above with reference to block 204, in some embodiments the target nutrient value for a nutrient type generated at block 204 may represent a recommended amount of the nutrient type to be consumed by the subject within a predetermined time period. In these embodiments, at block 206 the selection of the plurality of recommended food ingredients may be further based on at least one previously recommended food ingredient for the subject before the predetermined time period. For example, in some embodiments at least one previously recommended food ingredient may be stored in the memory 106 of the apparatus 100, and at block 204 the plurality of recommended food ingredients may be selected so as to avoid consecutively selecting or recommending the same or similar food ingredients that have been previously recommended during a predetermined time period. Therefore, a variety of food ingredients can be recommended to the subject, so as to increase likelihood of compliance of the subject to the meal plan and that a wide range of different nutrient types can be consumed by the subject.

In some embodiments, the selection of the plurality of recommended food ingredients at block 206 may comprise acquiring a plurality of candidate food ingredients from one or more databased based on the generated target nutrient value at block 204, and selecting a plurality of recommended food ingredients from the plurality of candidate food ingredients based on a user input. The plurality of candidate food ingredients may be displayed via the user interface 104 of the apparatus 100, and user input may also be received at the user interface 104. In these embodiments, the selection of the food ingredients may be manually performed by the user, instead of being performed by the processor 102 automatically.

Returning back to Fig. 2, at block 208, a meal plan is generated by selecting a recipe stored in one or more databases, based on the plurality selected recommended food ingredients at block 206. The selected recipe comprises a respective amount of each of a plurality of required food ingredients and food preparation instructions. In some embodiments, the selected recipe may comprise at least one required food ingredient matching one of the plurality of recommended food ingredients. The plurality of required food ingredients may include any type of food ingredients that is needed in order to prepare a course for a meal (e.g. a main course for dinner). The food preparation instructions may comprise cooking methods such as boiling, pan frying, oven baking, or other food preparation techniques such as cutting, peeling, and grating.

In some embodiments, more than one recipes may be selected. For example, for some meals more than one course may be served, for example dinner may include three courses. In this case, the meal plan may comprise at least one recipe for each of the course in the meal. Moreover, in some embodiments, the generated meal plan at block 208 may comprise more than one meals (e.g. comprising breakfast, lunch, and dinner). In this case, the meal plan may also comprise a plurality of recipes, wherein each of the plurality of recipes correspond to a course of a meal or a meal.

In some embodiments, the generated meal plan may comprise, in addition to the selected recipe, a time of day for the subject to prepare and/or consume the course or the meal corresponding to the selected recipe. In these embodiments, at block 208 the meal plan may be generated further on the basis a time of day and/or which meal (i.e. breakfast, lunch, afternoon tea, dinner, or snack) to which the selected recipe would correspond.

In some embodiments, at block 208 generating the meal plan may comprise acquiring a plurality of candidate recipes from the one or more databases based on the plurality of recommended food ingredients, and selecting one of the candidate recipes based on a user input. In these embodiments, each of the plurality of candidate recipes may comprise at least one required food ingredient matching one of the plurality of recommended food ingredients. The plurality of candidate recipes may be displayed via the user interface 104 of the apparatus 100, and user input may also be received at the user interface 104. In these embodiments, the selection of the recipe may be manually performed by the user, instead of being performed by the processor 102 automatically. Moreover, in some embodiments, an automatic selection of the recipe may be confirmed by a user via the user interface 104.

Returning back to Fig. 2, at block 210, a difference between a provided amount of the nutrient type by the selected recipe and the target nutrient value for the nutrient type generated at block 204 is determined. This determination may be performed by the processor 102, and is based on the amount of each of the plurality of required food ingredients of the selected recipe at block 208 and the food preparation instructions in the selected recipe. In embodiments where the generated meal plan comprises a plurality of recipes, a difference between a total provided amount of the nutrient type by the plurality of selected recipes and the generated target nutrient type may be determined. In some embodiments, the difference between the provided amount of the nutrient type by the selected recipe and the target nutrient value for the nutrient type may be represented by an absolute value or a percentage.

In some embodiments, at block 210 the determination of a difference between a provided amount of the nutrient type by the selected recipe and the generated target nutrient value for the nutrient type may be based on a retention rate of the nutrient type associated with at least one of the required food ingredients in the generated meal plan. The retention rate(s) of the nutrient type may be stored as a look-up table in the memory 106 of the apparatus 100.

In these embodiments, the memory 106 may store, for each of the plurality of required food ingredients in the selected recipe, a retention rate of at least one nutrient type associated with the required food ingredient. Moreover, in these embodiments, the retention rate may also be associated with the food preparation instruction. As an example, the retention rate of carbohydrate, protein, and fat of mutton when cooked with an oven are respectively 100%, 95%, and 80%. These retention rates can be used to calculate a provided amount of the nutrient type by the selected recipe. Therefore, the difference between this provided amount and the generated target nutrient value may be determined in cases where the generated meal plan comprises at least one recipe that has mutton as a food ingredient.

In some embodiments, the determination of the difference between the provided amount of the nutrient type by the selected recipe and the generated target nutrient value for the nutrient type may be based on data associated with a pre-processed state of at least one of the required food ingredients in the generated meal plan. For example, in cases where the selected recipe has mutton as a food ingredient, nutrient data relating to raw mutton may be used in the determination of the difference between the provided amount of the nutrient type by the selected recipe and the generated target nutrient value for the nutrient type. Moreover, in these embodiment, the provided amount of the nutrient type by the selected recipe may be calculated based on data associated with the pre-processed (e.g. raw) state of the food ingredient (e.g. beef) as well as a retention rate of the nutrient type (e.g. 95% retention of protein for mutton) when cooked/processed. Table 1 below contains nutrient data of raw and cooked mutton to demonstrate how the difference between the provided amount of the nutrient type and the generated target nutrient value for the nutrient type may be determined based on data associated with a pre-processed state of a required food ingredient:

**Table 1: nutrient data of raw mutton and cooked mutton**

| | Raw mutton (100g) | Raw mutton (300g) | Mutton after oven baking (based on 300g of raw mutton) | Raw mutton (360g) | Mutton after oven baking (based on 360g of raw mutton) |
|---|---|---|---|---|---|
| Energy (Kcal) | 285 | 855 | 715 | 1026 | 861 |
| Carbohydrates (g) | 4 | 11 | 11 | 14 | 14 |
| Protein (g) | 13 | 38 | 36 | 45 | 43 |
| Fat (g) | 24 | 73 | 59 | 88 | 70 |

Returning back to Fig. 2, at block 212, the meal plan generated at block 208 is adjusted based on the determined difference. This adjustment may be a change of at least one of: at least one of the plurality of required food ingredients in the selected recipe, the amount of at least one of the plurality of required food ingredients in the selected recipe, and the food preparation instructions in the selected recipe. In some other embodiments where the generated meal plan at block 208 comprises a time of day at which a course or a meal corresponding to the selected recipe is to be consumed by the subject, the adjustment of the meal plan may comprise adjusting the time at which the course or the meal is to be consumed. In some embodiments where the generated meal plan comprises a plurality of selected recipes, one of more selected recipe may be adjusted.

In some embodiments, the adjustment of the meal plan at block 212 may be performed so as to minimize the difference between the provided amount of the nutrient type by the selected recipe and the generated target nutrient value for the nutrient type.

To illustrate this with an example, the generated target nutrient for energy for the subject at block 204 may be 855 Kcal per meal and the provided amount of energy by the selected recipe at block 208 which contains mutton may be 715 Kcal. In this example, the determined difference is 140 Kcal and this difference can be minimized by increasing the amount of mutton in the recipe from 300 grams to 360 grams, according to Table 1 above. By increasing the amount of mutton in the recipe, the difference between the provided amount of energy (861 Kcal) and the generated target nutrient value for energy (855 Kcal) is reduced from 140Kcal to 6Kcal.

Although in the described example above the meal plan is adjusted by changing the amount of one of the required ingredients in the selected recipe, at block 212 the meal plan may be adjusted in one of many different ways to minimize the difference between the provided amount of the nutrient type by the selected recipe and the generated target nutrient value for the nutrient type. One way to minimize this difference may be to change at least one of the plurality of required food ingredients, for example in this instance to replace one of the required food ingredients in the selected recipe with a similar food ingredient that contains more energy (Kcal) per unit weight. Another way to minimize this difference may be to change an amount of the at least one of the plurality of required food ingredients, for example to increase an amount of at least one of the required food ingredients that contains a certain nutrient type. Another way to minimize this difference may be to change the food preparation instructions in the selected recipe, for example to replace a cooking technique with another cooking technique that causes less nutrient loss or to reduce the time of cooking.

In some embodiments, at block 212 the meal plan is adjusted if the determined difference between the provided amount of the nutrient type by the selected recipe and the generated target nutrient value for the nutrient type exceeds a predetermined threshold. In some embodiments, the predetermined threshold may be dependent on an accuracy requirement. In some embodiments, the predetermined threshold may be set by a user via the user interface 104.

For example, if it is determined at block 210 that the difference between the provided amount of the nutrient type and the generated target nutrient value is 16% and the predetermined threshold is 5%, then at block 212 the meal plan may be adjusted so as to minimize the difference. Conversely, if it is determined at block 210 that the difference between the provided amount of the nutrient type and the generated target nutrient value for the nutrient is 3% and the predetermined threshold is 5%, then at block 212 the meal plan may not be adjusted. Furthermore, in some of these embodiments, after adjusting the meal plan at block 212, the method may return to block 210 so as to determine a new difference between the provided amount of the nutrient type and the generated target nutrient value, and subsequently to block 212 where the meal plan may be further adjusted if the new determined difference still exceeds a predetermined threshold. In some of these embodiments, blocks 210 and 212 may be performed iteratively until the latest determined difference between the provided amount of the nutrient type by the selected recipe and the generated target nutrient value for the nutrient type does not exceed the predetermined threshold.

Although it is described above that the computer-implemented method comprises generating a target nutrient value for a subject for one nutrient type based on the acquired data, in alternative embodiments the computer-implemented method may comprise generating a target nutrient value for the subject for a plurality of nutrient types at block 204. For example, in these embodiments, at block 204, the processor 102 may be configured to generate a target nutrient value for a number of nutrient types including macronutrients and micronutrients, such as protein, carbohydrate, fat, vitamin C, magnesium, iron, etc. Also, in these embodiments, at block 206 the plurality of recommended food ingredients may be selected based on the plurality of target nutrient values for the plurality of nutrient types generated at block 204. Furthermore, in these embodiments, at block 210, a difference may be determined for at least one of the plurality of nutrient types, wherein the difference is between a provided amount of the respective nutrient type by the selected recipe and the target nutrient value for the respective nutrient type. Subsequently, at block 212, the meal plan may be adjusted based on at least one of these determined differences.

Although not illustrated in Fig. 2, the computer-implemented method in some embodiments may further comprise generating a respective amount to be consumed by the subject for each of the plurality of selected food ingredients. In these embodiments, at block 208 the selection of the recipe may be further based on at least one of the recommend amounts for the plurality of selected food ingredients. For example, at block 208 the selection of the recipe may be based on matching a generated amount of a selected food ingredient to be consumed by the subject with an amount of the same food ingredient required in a candidate recipe.

Although not illustrated in Fig. 2, the computer-implemented method may further comprise outputting the meal plan for the subject after it has been adjusted at block 212. The adjusted meal plan may be output via the user interface 104.

There is thus provided an improved method and apparatus for generating a personalized meal plan for a subject, which overcomes the existing problems.

There is also provided a computer program product comprising a computer readable medium, the computer readable medium having computer readable code embodied therein, the computer readable code being configured such that, on execution by a suitable computer or processor, the computer or processor is caused to perform the method or methods described herein. Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that maybe linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for generating a personalized meal plan for a subject, the method comprising:
acquiring (202) data associated with the subject;
generating (204) a target nutrient value for the subject for a nutrient type based on the acquired data;
selecting (206) a plurality of recommended food ingredients based on the generated target nutrient value;
generating (208) a meal plan by selecting a recipe stored in one or more databases based on the plurality of selected recommended food ingredients, wherein the selected recipe comprises a respective amount of each of a plurality of required food ingredients and food preparation instructions;
determining (210) a difference between a provided amount of the nutrient type by the selected recipe and the generated target nutrient value for the nutrient type, based on the amount of each of the plurality of required food ingredients and the food preparation instructions in the selected recipe; and
adjusting (212) the meal plan based on the determined difference.

2. A computer-implemented method according to claim 1, wherein adjusting (212) the meal plan comprising changing at least one of: at least one of the plurality of required food ingredients, the amount of at least one of the plurality of required food ingredients, and the food preparation instructions.

3. A computer-implemented method according to claim 1 or claim 2, wherein adjusting (212) the meal plan is performed so as to minimize the difference between the provided amount of the nutrient type and the generated target nutrient value for the nutrient type.

4. A computer-implemented method according to any of the preceding claims, wherein the meal plan is adjusted if the determined difference exceeds a predetermined threshold.

5. A computer-implemented method according to any of the preceding claims, wherein the target nutrient value represents a recommended amount of the nutrient type to be consumed by the subject within a predetermined time period, and selecting (206) the plurality of recommended food ingredients is further based on at least one previously recommended food ingredient for the subject before the predetermined time period.

6. A computer-implemented method according to any of the preceding claims, the method further comprising generating a respective recommended amount to be consumed by the subject for each of the plurality of selected food ingredients.

7. A computer-implemented method according to claim 6, wherein selecting the recipe is further based on at least one of the recommended amounts for the plurality of selected food ingredients.

8. A computer-implemented method according to any of the preceding claims, wherein generating (208) the meal plan comprises:
acquiring a plurality of candidate recipes from the one or more databases based on the plurality of recommended food ingredients; and
selecting one of the candidate recipes based on a user input.

9. A computer-implemented method according to any of the preceding claims, wherein selecting (206) the plurality of recommended food ingredients comprises:
acquiring a plurality of candidate food ingredients from one or more databases based on the generated target nutrient value; and
selecting a plurality of recommended food ingredients from the plurality of candidate food ingredients based on a user input.

10. A computer-implemented method according to any of the preceding claims, wherein determining (210) a difference between a provided amount of the nutrient type by the selected recipe and the generated target nutrient value for the nutrient type is based on a retention rate of the nutrient type associated with at least one of the required food ingredients in the generated meal plan.

11. A computer-implemented method according to any of the preceding claims, wherein determining (210) a difference between a provided amount of the nutrient type by the selected recipe and the generated target nutrient value for the nutrient type is based on data associated with a pre-processed state of at least one of the required food ingredients in the generated meal plan.

12. A computer-implemented method according to any of the preceding claims, wherein the data associated with the subject comprises information relating to at least one of: the gender of the subject, the age of the subject, the weight of the subject, the height of the subject, physical activity of the subject, a metabolic rate of the subject, a health target of the subject, and a food preference of the subject.

13. A computer-implemented method according to any of the preceding claims, wherein the nutrient type is one of: total energy, a macronutrient, a micronutrient.

14. A computer program product comprising a computer readable medium, the computer readable medium having computer readable code embodied therein, the computer readable code being configured such that, on execution by a suitable computer or processor, the computer or processor is caused to perform the method as claimed in any of the preceding claims.

15. An apparatus (100) for generating a personalized meal plan for a subject, the apparatus comprising a processor (102) configured to:
acquire data associated with the subject, wherein the acquired data comprises at least a food preference of the subject;
generate a target nutrient value for the subject for a nutrient type based on the acquired data;
select a plurality of recommended food ingredients based on the generated target nutrient value and the food preference of the subject;
generate a meal plan by selecting a recipe stored in one or more databases based on the plurality of selected recommended food ingredients, wherein the selected recipe comprises a respective amount of each of a plurality of required food ingredients, and food preparation instructions;
determine a difference between a provided amount of the nutrient type by the selected recipe and the generated target nutrient value for the nutrient type, based on the amount of each of the plurality of required food ingredients and the food preparation instructions in the selected recipe; and
adjust the meal plan based on the determined difference.
